(19) Europäisches Patentamt
European Patent Office
Office européen des brevets

(11) EP 1 841 372 B1

(12) # EUROPEAN PATENT SPECIFICATION

(45) Date of publication and mention of the grant of the patent:
**13.09.2017 Bulletin 2017/37**

(21) Application number: **06701706.1**

(22) Date of filing: **26.01.2006**

(51) Int Cl.:
***A61B 17/56*** (2006.01) ***A61B 17/88*** (2006.01)
***A61F 2/46*** (2006.01) ***A61F 2/36*** (2006.01)
***A61B 90/00*** (2016.01) ***A61B 34/20*** (2016.01)
*A61F 2/32* (2006.01) *A61F 2/34* (2006.01)
*A61B 17/15* (2006.01) *A61B 17/17* (2006.01)
*A61B 34/10* (2016.01) *A61B 34/00* (2016.01)

(86) International application number:
**PCT/CA2006/000107**

(87) International publication number:
**WO 2006/079211 (03.08.2006 Gazette 2006/31)**

(54) **COMPUTER-ASSISTED HIP JOINT RESURFACING METHOD AND SYSTEM**

COMPUTERGESTÜTZTES VERFAHREN UND SYSTEM ZUR HÜFTGELENKWIEDERHERSTELLUNG

PROCÉDÉ ET SYSTÈME DE RESURFAÇAGE DE L'ARTICULATION DE LA HANCHE ASSISTÉS PAR ORDINATEUR

(84) Designated Contracting States:
**AT BE BG CH CY CZ DE DK EE ES FI FR GB GR HU IE IS IT LI LT LU LV MC NL PL PT RO SE SI SK TR**

(30) Priority: **26.01.2005 US 646603 P**

(43) Date of publication of application:
**10.10.2007 Bulletin 2007/41**

(73) Proprietor: **Orthosoft Inc.**
**Montréal,**
**Québec H3C 2N6 (CA)**

(72) Inventors:
- **PARADIS, François**
  **Montréal, Québec H2S 2C3 (CA)**
- **FALARDEAU, Bruno**
  **Verdun, Québec H4G 2W2 (CA)**
- **JANSEN, Herbert, André**
  **Montréal, Québec H2H 3A6 (CA)**
- **AMIOT, Louis-philippe**
  **Hampstead, Québec H3X 3G8 (CA)**

(74) Representative: **Mays, Julie et al**
**Venner Shipley LLP**
**200 Aldersgate**
**London, EC1A 4HD (GB)**

(56) References cited:
**WO-A1-2004/032780 WO-A2-03/061501**
**WO-A2-2004/030556 WO-A2-2004/030556**
**CA-A1- 2 333 393 CA-A1- 2 439 850**
**CA-A1- 2 440 872 CA-A1- 2 482 006**
**DE-U1- 20 016 635 US-A- 5 769 092**

- **'BrainLAB, AG, BrainLAB orthopedic solutions Brochure - Product Portfolio: "VectorVision Hip SR - Reliable Hip Resurfacing"', [Online] 31 December 2005, XP008119913 Retrieved from the Internet: <URL:http://www.brainlab.com/download/pdf/OrthoCompleteBrochure.pdf>**
- **'BrainLAB AG, Brainwaves orthopedic solutions Newsletter Fall/Winter 2005/2006', [Online] 31 December 2005, XP008119920 Retrieved from the Internet: <URL:http://www.brainlab.com/download/pdf/brainwaves_orthopedics_fall05_.pdf>**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 841 372 B1

## Description

### FIELD OF THE INVENTION

The present invention generally relates to hip joint resurfacing surgery and, more precisely, to a method for assisting hip joint resurfacing surgery with computer-assisted surgery systems.

### BACKGROUND OF THE INVENTION

Hip joint resurfacing surgery involves the introduction of hip joint components in a patient. The acetabulum and the femoral head are resurfaced so as to receive an acetabular cup implant and a femoral head implant, respectively. The femoral head implant consists of a ball head received at an end of the resurfaced femoral head. Therefore, the implanted femoral head and the cup (i.e., acetabular or pelvic implant) coact to create the artificial hip joint. In comparison with total hip joint implanting surgery, the hip joint resurfacing surgery removes a relatively small amount of bone while providing high levels of joint stability.

Different output values are of concern in hip replacement surgery. In order to reproduce a natural and/or improved gait and range of motion to a patient, the position and orientation of the implants, the offset of the femur and the limb length must be considered during surgery. The work of the surgeon during hip replacement surgery will have a direct effect on these output values.

Known hip joint resurfacing surgery techniques presently involve specific tools so as to obtain precise position and orientation for the implants. As various types of reamers are used to resurface the femoral head, a plurality of alignment steps are performed to align the tools with the cuts to be made. It is, for instance, of nonnegligible importance that the femoral neck not be damaged (i.e., notched) by the reamers, to prevent fracture-prone weakness in the femoral head. Moreover, the resurfacing must be as precise as possible, for instance, to reduce the amount of cement required for implanting the ball head implant to the resurfaced ball head.

The document WO 2004/030556 describes a CAS system and method for guiding an operator in inserting a femoral implant in a femur as a function of a limb length and orientation of the femoral implant with respect to the femur, comprising a reference tool for the femur, a registration tool, a bone altering tool and a sensing apparatus.

Further, the document WO 03/061501 describes a method for intra-operatively presenting an approximate model of an anatomical structure by collecting a cloud of small surfaces. The cloud of small surfaces is gathered with a registration pointer having an adapted tip capable of making contact with the surface of an anatomical structure and registering the normal at the point of contact.

### SUMMARY OF THE INVENTION

The invention is defined in independent claims 1 and 10.

It is an aim of the present disclosure to provide a novel method for guiding an operator in inserting implants in hip joint resurfacing surgery.

It is a further aim of the present disclosure to provide a method of performing hip joint resurfacing surgery with computer assistance.

It is a still further aim of the present disclosure to provide a computer-assisted surgery system for guiding an operator in resurfacing bone surfaces in hip joint resurfacing surgery.

Therefore, there is provided a hip resurfacing CAS system for guiding an operator in altering a femoral head in computer-assisted surgery for subsequent implanting of a femoral head implant, comprising: a trackable reference on the femur, the trackable reference being trackable to form a femoral frame of reference of the femur; a registration tool being trackable; at least one bone-altering tool associated with a resurfacing of the femoral head, the at least one bone-altering tool being trackable; a tracking apparatus for tracking the trackable reference, the registration tool and the at least one bone-altering tool; and a resurfacing processing unit connected to the tracking apparatus so as to receive tracking data for the trackable reference, the registration tool and the at least one bone-altering tool, the resurfacing processing unit having a position/orientation calculator to calculate from the tracking data a position and orientation of the trackable reference to track the femoral frame of reference, and of the registration tool and the at least one bone-altering tool; a model generator receiving position and orientation data of the registration tool to produce a model of the femoral head and neck with respect to the femoral frame of reference; and a resurfacing evaluator determining an evaluated bone resurfacing alteration as a function of a position and/or orientation of the at least one bone-altering tool with respect to the bone model of the femoral head and neck, and a tool geometry model, at least prior to resurfacing being performed.

Further there is provided a method of doing surgical treatment with a tracking apparatus in computer-assisted surgery for guiding an operator in resurfacing a femoral head for a subsequent implanting of a femoral head implant, comprising the steps of: defining a frame of reference of the femur, the frame of reference being trackable in space for position and orientation; producing a model of a femoral head and neck with respect to the frame of reference; selecting an orientation of a bone-altering tool with respect to the model of the femoral head and neck as a function of an evaluated bone resurfacing alteration varying with said orientation of the bone-altering tool; and creating a guide channel in the femoral head with the bone-altering tool in the selected orientation, for subsequent resurfacing of the femoral head.

BRIEF DESCRIPTION OF THE DRAWINGS

These and other features, aspects and advantages of the present invention will become better understood with regard to the following description and accompanying drawings wherein:

Fig. 1 is a front elevation view of leg bones involved in a hip replacement method in accordance with the present disclosure;
Fig. 2 is a flowchart of a method for hip joint resurfacing surgery in accordance with a preferred embodiment of the present disclosure; and
Fig. 3 is a view of a user interface illustrating the Step 120 of selecting a guide orientation; and
Fig. 4 is a block diagram of a hip resurfacing CAS system in accordance with another preferred embodiment of the present disclosure.

DESCRIPTION OF THE PREFERRED EMBODIMENT

According to the drawings, and more particularly to Fig. 1, bones of the leg that will be involved in the hip replacement surgery of the present disclosure are generally shown at 1. Fig. 1 is provided as reference for the description of the steps of the hip replacement surgery method described herein. The bones are the pelvis 10, the femur 20, the tibia 30 and the fibula 40. Hereinafter, parts of these bones will each be referenced by numerals from the same numeric decade. For instance, parts of the pelvis (e.g., the acetabulum 11) will bear reference numerals between 11 and 19.

Referring to Fig. 2, a method for hip joint resurfacing surgery in accordance with the present disclosure is generally shown at 100. Although the method 100 is referred to in the singular, various choices of procedure will be given to the surgeon, as will be set forth in the forthcoming description, according to the preferences of the surgeon. A plurality of methods can be derived from the method 100 according to the decisions of the surgeon.

In Step 102, preparative steps for surgery are effected. Namely, general patient information can be entered into a CAS system for opening a patient file. For instance, a general patient profile can be entered, consisting of the name, birth date, identification number, sex and the like, the side to be operated, as well as more specific data pertaining to the surgery, such as leg length discrepancy (with the identification of the longer leg), if applicable, and parameters to define the flow of the application and the display. For instance, the leg length discrepancy is measured using X-rays of the hip joint. More precisely, the leg length discrepancy is measured from the vertical comparison between the lesser trochanters. These X-rays are typically taken during the diagnostic stages leading to surgery, so they are usually available for hip joint surgery. Alternatively, X-rays may be taken as part of Step 102. It is also contemplated to import DICOM files or digital X-rays.

It is pointed out that the general patient information can be entered preoperatively. Moreover, the entering of the general patient information is straightforward such that the surgeon need not be involved. However, in order to minimize the preoperative procedures, actions of Step 102 can be performed at the beginning of the surgical session, during the short time span preceding the surgery.

Other values that will potentially be considered in the method 100 are inclination and anteversion for the pelvic implant, CCD and anteversion for the femoral implant

The calibration of the various surgical tools to be used is done. For instance, a calibration base and method, as set forth in International Publication No. WO 01/67979 A1 by Jutras et al., can be used for the calibration. Also, correspondence between the tracking of the tools and the display on a CAS system can be verified in further calibration steps included in Step 102. A permanent calibration system can also be used, as set forth in International Publication No. WO 2005/102202.

Surgery is initiated between Step 102 and subsequent Step 104, by the surgeon exposing the hip joint. No computer assistance is required thereat.

In Step 104, the trackable references are secured to the pelvis with a pelvic modular reference, and to the femur with a femoral modular reference. The pelvic modular reference can be inserted in a cranial or lateral position. Alternatively, the trackable references may be secured prior to exposing the hip joint.

It is pointed out that the pelvic modular reference, in a preferred embodiment, must be positioned while the patient is in supine decubitus. Moreover, as will be described hereinafter, the pelvic coordinate system and table reference must also be digitized in supine decubitus. After those manipulations, the patient can be repositioned in lateral decubitus.

The femoral modular reference can be inserted at the proximal third from the femoral head of the femur or at the distal third from the femoral head. These insertion points are examples, as any other suitable point on the femur is considered. Positions of the trackable references are, for example, (1) looking posterior and towards the head, prior to dislocation, and (2) a longer trackable reference, looking posterior, for the dislocated position. It is contemplated to use a single modular base.

In Step 106, it is contemplated to digitize the coordinate system in lateral decubitus. It is also contemplated to collect posture information, as described in International Publication No. WO 2004/030559 A1, by Jansen et al. Criteria may be used to validate the points taken and the computed surface.

In Step 106, a pelvic coordinate system and a femoral coordinate system are digitized. The pelvic coordinate system is digitized with a registration pointer. In an embodiment, three points are taken on the pelvis 10 to create the frontal plane of the acetabular coordinate system. Referring to Fig. 1, there is one point on the iliac crest 12 of the operated side, one point on the contra lateral iliac crest 13, and one point on one of the two pubic tubercles 14 of the pelvis 10. To be generally aligned, the points digitized on the iliac crests 12 and 13 are taken at the outermost anterior point of the iliac crests 12 and 13. The points digitized on the iliac crests 12 and 13 are preferably taken directly on the soft tissue covering the bone pelvis on the iliac crests, as the soft tissue is relatively thin thereon. The point on the pubic tubercle 14 completes a first plane, the frontal plane. A second plane, the transverse plane, is perpendicular to the frontal plane and includes the points on the iliac crests. A third plane, the sagittal plane, is perpendicular to the frontal and transverse planes.

Supplemental information regarding the frontal plane can be obtained for various postures of a patient. For instance, trackable references can be used to gather information about sitting, standing and walking postures. This information can be used to adjust the orientation of the frontal plane, as these postures can provide information not available from the typical lying posture in which a patient is during surgery. This information can influence the anteversion positioning of the implants.

It is possible to obtain anteversion and/or inclination values of the acetabulum of the patient, to be used as a reference (e.g., comparison basis) later in the surgery. To do so, points are digitized using a registration pointer on the generally circular edge of the acetabulum 11 and a plane is defined from these points. A normal to this plane and the pelvic frontal plane give the anteversion angle. The normal to this plane is projected onto the acetabular frontal plane to give an inclination angle with a cranial-caudal axis.

For the digitization of the femoral coordinate system, it is contemplated to collect five points of reference on the leg to the computer assisted surgery system, which is equipped with software that will create the femoral coordinate system.

Referring to Fig. 1, a first point is taken on the tip of the greater trochanter 23 of the femur 20, and will be defined as a starting point of an anatomical axis of the femur 20. Thereafter, points are taken on the medial and lateral epicondyles 24 and 25 of the femur 20, respectively. A midpoint between the medial epicondyle and lateral epicondyle points, in alignment therewith, is defined as an endpoint of the anatomical axis of the femur. The fourth and fifth points are taken on the medial malleolus 31 of the tibia 30 and on the lateral malleolus 41 of the fibula 40, with the leg being bent at the knee. By having the leg bent at the knee, the tibia 30 stands on the posterior condyles 26 of the femur 20. Therefore, an assumption is made wherein an aligned midpoint of the medial and lateral malleoli points is said to define a plane (i.e., sagittal plane) with the anatomical axis, with an axis of the knee being normal to the sagittal plane. The frontal plane is perpendicular to the sagittal plane, with the anatomical axis lying therein. The transverse plane is perpendicular to the sagittal and frontal planes, and can be positioned at any height. With the anatomical axis and the midpoint of the malleolus region digitized, the femoral coordinate system, i.e., the femoral frame of reference, is complete. It is noted that it is not required to measure two points to obtain a midpoint of the malleolus region. As this latter point will be in the sagittal plane, the only requirement is that a point is taken at a midpoint of the malleolus region, and may thus be placed approximately by the operator.

It is pointed out that the projection values described herein (e.g., inclination, anteversion, etc.) are based on the acetabular and the femoral coordinate systems. As it is contemplated to use alternative methods of digitizing the acetabular and the femoral coordinate systems, in addition to the preferred methods of Step 116, the projection values would be related to the alternative acetabular and femoral coordinate system.

Other methods to gather information pertaining to surgical parameters are as follows. (1) The user digitizes a point on the greater trochanter before dislocation and retakes the same point, with the leg aligned in the same orientation, after reduction. (2) The user digitizes a point on the greater trochanter before dislocation and the system helps the user to replace the leg in the same orientation after reduction. The leg length and the offset are automatically computed when the leg is positioned in range of the initial position before dislocation. (3) The user digitizes many points near the greater trochanter before dislocation, the center of rotation of the acetabulum as described in Step 112 and the same points after reduction. The system aligns these points and computes the leg length and the offset. Also, in each case, the CAS system may help the operator in placing the leg in a required initial position.

In optional Step 108, a relative position between the pelvis and the femur is registered with respect to the trackable references. The leg is simply left in a straight position, to align with a longitudinal axis of the body, and a relative position is acquired between tracking references secured to their respective bones.

In Step 110, the femur is dislocated from the pelvis, so as to expose the acetabulum 11 and the femoral head 21 and neck 22.

In Step 112, a center of rotation is digitized for the acetabulum, by taking reference points on the surface of the acetabulum 11. A center calculator (e.g., sphere fitter algorithm) is used to find the acetabular center of rotation, and will be described hereinafter with the description of a hip resurfacing CAS system. The acetabular center of rotation is therefore known as a function of the tracking reference on the pelvis 10. In order to ensure precise results, it may be required that a predefined number of points be taken until validation criteria are met. Visual validation of the sphere found by the algorithm can also be performed. The center of rotation and the diameter found may be displayed. Points are digitized in the fossa (depth of the acetabulum). Points are displayed by small spheres or disks (many colors possible). If the center of rotation of the acetabulum is known, it is not necessary to digitize the center of rotation of the femoral head.

The registration of points in the acetabulum can also be taken by a real-time tracing of the acetabulum surface (i.e., painting the acetabulum surface). In this case, points can be used to build a mesh. The mesh can be constructed while points are acquired so the user may take more points when needed to have a more precise reconstruction. Criteria may then be used to validate the points taken and the computed center of rotation and diameter.

In Step 114, the acetabulum is altered in view of accommodating the acetabular cup implant. In order to guide the operator in altering the acetabulum, reamer position and orientation information is preferably provided, such that an axis of actuation of the reamer is for instance visually displayed. The previous acetabular center of rotation is known as a function of the tracking reference secured to the pelvis 10, as it was acquired in previous Step 112. Preferably, the reamer is tracked for position and orientation.

Examples of information that can be provided to the operator are as follows: generic 2D images, mosaic or mesh in 3D viewers along with drive shaft/reamer assembly in real time and/or display targeting views to help the user to align with target values, frontal and lateral views, inclination, inclination adjusted with the pelvic tilt, anteversion, anteversion adjusted with the pelvic tilt angles in real time, 3D position of the reamer center of rotation relatively to the acetabulum center of rotation, the distance between the reamer pole and acetabular wall.

The diameter of the pelvic implant chosen by the surgeon can be used to display a position of the new acetabular center of rotation in comparison to the digitized acetabular center of rotation (Step 112). For instance, the distance between the centers of rotation can be displayed numerically (e.g., in mm) as a function of the acetabular coordinate system digitized in previous Step 106. Also, the anteversion and inclination of the actuation axis of the reamer, both as a function of the acetabular coordinate system, can be given numerically (e.g., in degrees) to guide the surgeon in the reaming. More precisely, the anteversion is calculated as the angle between the axis of the reamer and the pelvic frontal plane, and the inclination is the angle between the reamer axis projected onto the acetabular frontal plane and a cranial-caudal axis (Step 106).

Step 116 consists in the insertion of the pelvic implant in the acetabulum 11, but it is pointed out that this step can also be performed once the femoral head implant has been secured to the femur, according to the preference of the operator. A tracked impactor is preferably used. As the pelvic implant size is known, the diameter thereof and the known relation between the impactor and the pelvic implant is used with the tracking of the impactor to give the anteversion and the inclination of the pelvic implant. Also, the distances between the current and the digitized centers of rotation can be displayed. Therefore, the surgeon is guided during the use of the impactor so as to position the pelvic implant to a given position of the center of rotation thereof, and to a given orientation [with respect to anteversion and inclination] to provide a maximal range of motion and stability of the leg.

Although the pelvic implant is secured at this point to the pelvis 10, it is possible to adjust the position and orientation of the pelvic implant. Firstly, the tracked impactor, handle or like tool may be reconnected to the pelvic implant to serve as a lever in manipulating the pelvic implant with the tracked impactor, allowing position and orientation information (e.g., anteversion and inclination) to be calculated from the tracking of the impactor. Alternatively, points on the circular edge of the pelvic implant may be digitized to define a plane, with the normal to this plane being used to calculate the anteversion and the inclination, as suggested previously to obtain this information for the acetabulum.

Information typically provided with the use of the impactor includes: Display of generic 2D images, mosaic or mesh in 3D viewers along with impactor/cup assembly in real time and/or display targeting views to help the user to align with target values, frontal and lateral views, navigation of the impactor and cup, display of inclination, inclination adjusted with the pelvic tilt, anteversion, anteversion adjusted with the pelvic tilt angles in real time, display of the 3D position of the cup center of rotation relatively to the acetabulum center of rotation.

In Step 118, a bone model is digitized for the femoral head 21 and neck 22. A registration pointer having its tip tracked in space is used to register points on the surface of the femoral head 21 and neck 22. Therefore, points of contact between the tip and a given surface can be registered as a function of the tracking reference (Step 104). As tracking references have been secured to the femur 20 and the pelvis 10 in Step 104, the points on the surface of the femoral head 21 are known as a function of the tracking of the respective tracking reference of the femur 20. As will be described hereinafter, a digital model of the femoral head and neck is produced, and may be displayed visually by the hip resurfacing CAS system.

It is pointed out that the neck/head connection is identified in the digital model of the femoral head and neck. Information preferably obtained includes the lateral aspect of femur at the greater trochanter and the following 10 cm distally (as far as possible), internal aspect of femur at the lesser trochanter and the following distal region, and femoral neck itself (varus/valgus, anteversion). The head-neck junction is digitized or computed based on the points taken. If points are acquired automatically, collection of points can be taken by painting the femur. If points are acquired to build a mesh, points are taken on all the surface of the femur and not only on the frontal and transverse plane. The mesh can be constructed while points are acquired so users may take more points to have a more precise reconstruction.

The center of rotation of the femoral head may also be calculated from the digital model, for instance using a sphere fitter algorithm. If the center of rotation of the acetabulum is known, it may not be necessary to digitize the center of rotation of the femoral head.

In Step 120, the desired guide orientation is determined. More specifically, the resurfacing of the femoral head will be dependent on the orientation of the guide wire. Therefore, computer assistance is provided to the operator so as to orient the guide wire in view of the subsequent resurfacing of the femoral head.

In order to plan the orientation of the guide wire, various views are provided such as the frontal and top views of the reconstructed femur. A template of the femoral implant over the femur model is also provided, as well as the following information: the initial CCD and anteversion angles, an initial template position, orientation and size with respect to the femoral center of rotation. The CCD is calculated as the angle between the projection of the guide wire on the femoral frontal plane and the longitudinal axis of the femur. Widgets are provided on screen to translate and rotate the template in each view. Selectors are provided to set the size of the implant and the neck diameter. The neck diameter is found by two moving lines parallel to the template axis. When the lines are on the contour of the neck, the diameter is determined. The CCD and anteversion angles are computed and displayed while the user is positioning the template. It is also contemplated to provide means to rotate the model so it can be viewed in 360 degrees. Implant position, orientation and size are computed and suggested to the operator as information to consider. Information that is preferably computed and displayed includes: The estimated range of motion, the estimated final leg length and offset, a graphical representation of the femoral preparation (final result). Potential dislocation and/or impingement is identified based on the cup position and orientation and the planned position and orientation of the femoral implant. If the femur is reconstructed with a mesh, the percentage of coverage may be provided. Indications of where notching may happen should also be provided.

In Step 122, the femur is altered for the insertion of the guide wire. In order to guide the operator in positioning and orienting the guide wire as planned, various information is provided, such as: Generic 2D images, mosaic or mesh in 3D viewers along with guide wire/drill guide in real time and/or display targeting views to help the user to align with planned values, frontal and top views of the reconstructed femur, navigation of the guide wire with a drill guide, the CCD and anteversion angles, alignment views of the guide wire tracked with the drill guide on the CCD and anteversion axis found during the planning phase (aligning "bull's-eyes" or axes), the CCD and anteversion angles of the guide wire, audio and/or visual cues to let the operator know he/she is "in range" near the targeted angles by the means, the depth of the guide wire so the operator will be able to determine when the tip of the guide wire is near the lateral cortex of the proximal femur, potential notching with audio and/or visual feedback, and where this notching could potentially occur. An example is provided in Fig. 3, in which a drill guide axis is oriented as a function of the bone model.

The same information can be provided for the insertion of a cannulated drill guide, with a display of the depth of drilling so the user will be able to determine when to stop drilling according to the chosen implant size.

Haptic devices can be used to ensure that the drilling only occurs when the orientation of the guide wire is as planned.

In Step 124, the femoral head 21 is resurfaced, by way of a reamer. It is contemplated to provide visual information to the operator at this step. However, the guides inserted in the femur ensure that the reaming follows planning. It is preferred that the operator keeps inspecting the actual femur especially during the cylindrical reaming, so as to avoid notching of the femoral neck 22. Information that can be provided is as follows: Tracking for position and orientation of the cylindrical reamer, generic 2D images, mosaic or mesh in 3D viewers along with cylindrical reamer in real time, frontal and top views of the reconstructed femur, navigation of the cylindrical reamer to track the reamed depth, orientation and position, the CCD and anteversion angles, a graphical representation of the result of the reaming, a pre-notching warning system based on probability to notch the cortex when the instrument is close to it.

For the planar reaming, information that can be provided is as follows: Generic 2D images, mosaic or mesh in 3D viewers along with planar reamer in real time, frontal and top views of the reconstructed femur, tracking of the planar reamer to track the reamed depth, orientation and position, the CCD and anteversion angles, the distance between the head-neck junction and the plane surface of the planar reamer, indications to the operator to stop reaming based on the selected implant size, how much bone has been removed, the leg length and the offset based on the position of the planar reamer, a graphical representation of the result of the reaming, pre-notching warning system based on probability to notch the cortex when the instrument is close to it.

In Step 126, the femoral implant is secured to the resurfaced femoral head. Information that can be provided is as follows: position and orientation of the femoral component, generic 2D images, mosaic or mesh in 3D viewers, frontal and top views of the reconstructed femur, navigation of the cement mantel to track the position and the orientation of the implant, the distance between the implant and the plane surface of the femur, the leg length and the offset. It is contemplated to provide the possibility to attach the femoral implant while in place.

Although not illustrated in the method, there is provided the possibility to ream again the acetabulum after the placement of the femoral component if the initial reaming is not adequate, following the options provided in Step 114. Also, Step 116 could be performed at this point. Information that can be provided includes: the leg length and the offset based on the position of the reamer relatively to the acetabulum center of rotation and the position and orientation of the femoral implant with respect to the femur.

In the event that the acetabular cup is implanted at this point, the information that can be provided is as follows: Tracking of the cup impactor, generic 2D images, mosaic or mesh in 3D viewers along with impactor/cup assembly in real time and/or display targeting views to help the user to align with target values, frontal and lateral views, display inclination, inclination adjusted with the pelvic tilt, anteversion, anteversion adjusted with the pelvic tilt angles in real time, 3D position of the cup center of rotation relatively to the acetabulum center of rotation, the leg length and the offset based on the position of the impactor relatively to the acetabulum center of rotation and location of the femoral component on the femur.

In Step 128, an analysis of range of motion is performed. Information is calculated, such as the range of motion of the joint after reduction, inclination, rotation and flexion/extension, possible dislocation (i.e., detect if the center of rotation has moved) and/or impingement.

Referring to Fig. 4, a hip resurfacing CAS system is generally shown at 200. The CAS system 200 has a resurfacing processing unit 201. The resurfacing processing unit 201 is typically a computer or like device having a processor.

Peripherals are provided in association with the resurfacing processing unit 201. In view of the trackable references 202 that will be secured to the femur and pelvis to define frames of reference (Steps 104 and 106) and to the tracked tools 204 used throughout the method 100, tracking apparatus 206 is connected to the processing unit 201. The tracking apparatus 206 is provided to track the trackable references 202 and the tools 204 in the selected surgical environment. The tracking apparatus 206 may be any of optical sensors, RF sensors, magnetic sensors and the like used in CAS systems.

Interface 207 is connected to the processing unit 201. The interface 207 enables data entry and communications from the operator/surgeon of the system 200 to the processing unit 201. For instance, the interface 207 may be a keyboard, mouse and/or touch screen or the like.

A display unit 208 is connected to the processing unit 201. The display unit 208 provides information to the operator/surgeon throughout the steps of the method 100. The data may be in the form of numerical values, as well as virtual representations of bone models along with simulations of tools. Further detail about the data displayed by the display unit 208 will be given hereinafter.

The resurfacing processing unit 201 has a computer-assisted surgery controller 210. The CAS controller 210 is connected to the tracking apparatus 206 and to the interface 207, so as to receive information therefrom. More specifically, the CAS controller 210 receives tracking data from the tracking apparatus 206, which tracking data will be interpreted by the processing unit 201. The CAS controller 210 receives user commands given by the operator of the system 200 using the interface 207, and essentially controls the flow of information between the peripherals 206 to 208, and between the other components 212, 214, 216, and 218 of the resurfacing processing unit 201. The CAS controller 210 performs certain tasks as well, such as calibration of tools.

The CAS controller 210 is also connected to the display unit 208. The CAS controller 210 provides display data, in the form of numerical values and visual representations, to the display unit 208. The display unit 208 displays this information.

A position/orientation calculator 212 is connected to the CAS controller 210. The position/orientation calculator 212 receives the tracking data of the tracking apparatus 206 from the CAS controller 210. The information provided to the CAS controller 210 by the position/orientation calculator 212 is in the form of the position/orientation of a selected item of the trackable references 202 or tools 204. For instance, following the method 100, the data provided by the calculator 212 may be the pelvic and femoral coordinate systems from the trackable references 202. As another example, the data takes the form of a real-time orientation of the operating axis of one of the tools 204, such as the axis of a reamer, or a real-time position of a tip of one of the tools 204, such as a registration pointer.

A center calculator 214 is associated with the CAS controller 210. The center calculator 214 is provided to digitize the center of rotation of the pelvis (as described for Step 112) and the center of rotation of the femoral head (optionally in Step 118). The center calculation is performed using the position/orientation data calculated by the position/orientation calculator 212, as well as commands from the CAS controller 210.

For instance, the center calculation is performed using the tracked position of a registration pointer/tool from the tools 204, pointing or brushing the surface of the acetabulum (Step 112) or of the femoral head (Step 118). An indication that the center calculation is to be performed by the center calculator 214 is commanded by the CAS controller 210, for instance as a response to a command from the operator using the interface 207. The position of the centers is therefore calculated with respect to the coordinate systems (Step 106), and the information is updated in real-time by the CAS controller 210.

A model generator 216 is associated with the CAS controller 210. The model generator 216 receives position/orientation data in combination with commands from the CAS controller 210, following Steps 112 and 118. For instance, points registered by a registration pointer/tool or the like from amongst the tools 204 are used to construct a bone surface model. As discussed previously, a bone model may be generated prior to surgery, whereby the model generator 216 is provided to calibrate the bone model with the femoral frame of reference. For instance, in Step 118, a surface model of the femoral head and neck is obtained. The surface model is associated with the coordinate systems obtained from the tracking of the trackable references 202.

A resurfacing evaluator 218 is provided in association with the CAS controller 210. The resurfacing evaluator 218 is provided to determine the evaluated bone resurfacing alteration, which is the effect of a resurfacing tool (from the tools 204) on the bone model. Accordingly, bone model data is provided by the model generator 216, along with the position and orientation of a reaming tool as determined by the CAS controller 210 from tool geometry data and an orientation of a bone-altering tool (such as a drill) from the tools 204.

In the case of femoral head resurfacing, as the precision of the reaming must be respected, it has been described previously that a guide wire is provided, in order to drill a guiding bore in the femoral head prior to reaming. Therefore, the evaluated bone resurfacing alteration is indicated as a function of the orientation of the axis of the drill guide. Therefore, information associated with a potential wrongful reaming is provided to the operator, such that the operator is guided into drilling the drill guide in a suitable orientation in view of the effects on resurfacing. The resurfacing evaluator 218 may also be used to calculate the effect of acetabulum reaming on associated data (pelvic center of rotation, anteversion, etc.)

Throughout surgery, the display unit 208 provides the data discussed above. For instance, the output of the model generator 210 is converted by the CAS controller 210 to a virtual model of the bone surface to be altered, for instance with virtual real-time representations of the tools with respect to the bone models. Accordingly, warning can be signaled to the operator/surgeon if the effects of resurfacing are outside acceptable standards. Again, in femoral head resurfacing, the femoral neck must not be nicked, whereby drill guide axis data can be associated with a warning signal to guide the operator/surgeon in adjusting the orientation of the drill.

Moreover, numerical information is also provided to the operator, which numerical information is described previously for the steps of the method 100.

Various instruments can be used, such as blunt tracked pointers (straight or curved), adapted to fit on a rotational tracker or a universal handle to paint bones (acetabulum, femur, etc.). The drill guide or guides can be designed to fit on a universal handle or a rotational tracker. A mechanism may be used to block/hold the position and the orientation of the drill guide. Planar reamer is modified to be used in conjunction with the rotational tracker. Technology to have appropriate drilling instrument if the user wants to navigate the drill bit only.

In other contemplated options there are the possibility to navigate the guide wire, the guide wire and the cannulated drill bit or only the drill bit, the possibility to rotate, translate and zoom the viewers, the animation or illustration to describe to the operator the upcoming tasks, the possibility to take snapshots, menus allowing selection of options and parameters during the procedure, allowing navigating through the surgical steps in the application, step-driven (wizardlike sequence of pages), status icons to display tracking state of an instrument, volume view/aim camera to display in space the location of the trackers seen by the camera, give information on the tracked state of a tracker (out of volume, missing sphere, IR interference, etc).

A calculation that can be performed and provided as information to the operator is the femoral target height. The target height is a desired position for the femoral center of rotation, and is calculated as follows:

$$(\text{target height}) = (\Delta_{PELVIC\ COR}) - (\text{initial } \Delta_{LL}),$$

where $(\Delta_{PELVIC\ COR})$ is the deviation of the implant center of rotation with respect to the digitized acetabular center of rotation, in cranial-caudal (y) direction (with a cranial deviation having a positive value), and $(\text{initial } \Delta_{LL})$ is the initially acquired limb length discrepancy.

## Claims

1. A hip resurfacing CAS system (200) for guiding an operator in resurfacing a femoral head in computer-assisted surgery for subsequent implanting of a femoral head implant, comprising:

    a trackable reference (202) on the femur, the trackable reference being trackable to form a femoral frame of reference of the femur;
    a registration tool (204) being trackable;
    at least one bone-altering tool (204) associated with a resurfacing of the femoral head, the at least one bone-altering tool being trackable;
    a tracking apparatus (206) for tracking the trackable reference (202), the registration tool (204) and the at least one bone-altering tool (204); and
    a resurfacing processing unit (201) connected to the tracking apparatus (206) so as to receive tracking data for the trackable reference (202), the registration tool (204) and the at least one bone-altering tool (204), the resurfacing processing unit having:

        a position/orientation calculator (212) to calculate from the tracking data a position and/or orientation of the trackable reference (202) to track the femoral frame of reference, and of the registration tool (204) and the at least one bone-altering tool (204);
        a model generator (216) receiving position and/or orientation data of the registration tool (204) to produce a model of the femoral head and neck with respect to the femoral frame of reference; and
        a resurfacing evaluator (218) determining an evaluated bone resurfacing alteration of the femoral head as a function of a position and/or orientation of the at least one bone-altering tool (204) with respect to the bone model of the femoral head and neck, and a tool geometry model, at least prior to resurfacing being performed.

2. The hip resurfacing CAS system (200) according to claim 1, wherein the at least one bone altering tool (204) is a drill provided to perform a guide channel in the femoral head to subsequently guide a resurfacing tool, an orientation of the drill being used by the resurfacing evaluator to determine the evaluated bone resurfacing alteration by the resurfacing tool as a function of the tool geometry model of the resurfacing tool.

3. The hip resurfacing CAS system (200) according to claim 1, further comprising a display unit connected to the resurfacing processing unit to visually provide data associated with the evaluated bone resurfacing alteration.

4. The hip resurfacing CAS system (200) according to claim 3, wherein the data associated with the evaluated bone resurfacing alteration is a real-time visual representation of the tool geometry model as oriented with respect to the model of the femoral head and neck.

5. The hip resurfacing CAS system (200) according to claim 4, wherein the at least one bone altering tool (204) is a drill provided to perform a guide channel in the femoral head to subsequently guide a reaming tool, an orientation of the drill being used by the resurfacing evaluator to determine the evaluated bone resurfacing alteration by the reaming tool as a function of the tool geometry model of the reaming tool, the reaming tool geometry model being illustrated as parallel lines oriented with respect to the model of the femoral head and neck.

6. The hip resurfacing CAS system (200) according to claim 1, wherein the resurfacing processing unit (201) has a surgical parameter calculator to calculate surgical parameters with respect to the femoral frame of reference, as a function of tracking data at least one of the registration tool (204), the at least one bone-altering tool (204), and additional surgical tools tracked for position and orientation.

7. The hip resurfacing CAS system (200) according to claim 6 , wherein the surgical parameters include any one of the position of the center of rotation of the femoral head, varus/valgus angle, anteversion angle, limb length discrepancy.

8. The hip resurfacing CAS system according to claim 1, wherein the model generator produces the model of the femoral head and neck by obtaining a predetermined amount of points on the femoral head and neck by the tracking of the registration tool.

9. The hip resurfacing CAS system according to claim 1, wherein the resurfacing processing unit (201) comprises a pre-notch warning system so as to avoid notching of the femoral neck.

**10.** A method of doing surgical treatment with a hip resurfacing CAS system according to any of claims 1-9 for guiding an operator in resurfacing a femoral head for a subsequent implanting of a femoral head implant, wherein the method is performed on an anatomical bone model or on a cadaver, the method comprising the steps of:

defining (104, 106, 108) a frame of reference of the femur, the frame of reference being trackable in space;
producing (118) a model of a femoral head and neck with respect to the frame of reference;
selecting (120) an orientation of a bone-altering tool with respect to the model of the femoral head and neck as a function of an evaluated bone resurfacing alteration of the femoral head varying with said orientation of the bone-altering tool; and
creating (122) a guide channel in the femoral head with the bone-altering tool in the selected orientation, for subsequent resurfacing of the femoral head.

**11.** The method according to claim 10, wherein the model of the femoral head and neck is produced by digitizing a surface of the femoral head and neck.

**12.** The method according to claim 10, wherein the evaluated bone resurfacing alteration is a real -time visual representation of a geometry of a resurfacing tool with respect to the model of the femoral head and neck.

**13.** The method according to claim 10, further comprising a step of obtaining surgical parameters when selecting the orientation of the bone-altering tool and when creating the guide channel.

**14.** The method according to claim 13, wherein the surgical parameters include any of the position of the center of rotation of the femoral head, varus/valgus angle, anteversion angle, limb length discrepancy.

**15.** The method according to claim 10, wherein creating (122) a guide channel in the femoral head comprises warning where potential notching of the femoral neck could occur.

**Patentansprüche**

**1.** Computergestütztes System zur Hüftgelenkwiederherstellung (200) zum Führen eines Operateurs beim Wiederherstellen eines Oberschenkelkopfes bei computergestützter Chirurgie zum anschließenden Implantieren eines Oberschenkelkopfimplantats, umfassend:

eine verfolgbare Referenz (202) auf dem Oberschenkel, wobei die verfolgbare Referenz verfolgbar ist, um einen Oberschenkelreferenzrahmen des Oberschenkels zu bilden;
ein Registrierungswerkzeug (204), das verfolgbar ist;
mindestens ein Knochenänderungswerkzeug (204), das mit einem Wiederherstellen des Oberschenkelkopfes assoziiert ist, wobei das mindestens eine Knochenänderungswerkzeug verfolgbar ist;
eine Verfolgungsvorrichtung (206) zum Verfolgen der verfolgbaren Referenz (202), des Registrierungswerkzeugs (204) und des mindestens einen Knochenänderungswerkzeugs (204); und
eine Wiederherstellungs-Verarbeitungseinheit (201), die mit der Verfolgungsvorrichtung (206) verbunden ist, um Verfolgungsdaten für die verfolgbare Referenz (202), das Registrierungswerkzeug (204) und das mindestens eine Knochenänderungswerkzeug (204) zu empfangen, wobei die Wiederherstellungs-Verarbeitungseinheit Folgendes aufweist:

einen Positions-/Orientierungskalkulator (212) zum Berechnen, aus den Verfolgungsdaten, einer Position und/oder Orientierung der verfolgbaren Referenz (202) zum Verfolgen des Oberschenkelreferenzrahmens, und des Registrierungswerkzeugs (204) und des mindestens einen Knochenänderungswerkzeugs (204);
einen Modellgenerator (216), der Positions- und/oder Orientierungsdaten des Registrierungswerkzeugs (204) empfängt, um ein Modell des Oberschenkelkopfes und -halses mit Bezug auf den Oberschenkelreferenzrahmen zu erzeugen; und
einen Wiederherstellungsauswerter (218), der eine ausgewertete Knochenwiederherstellungsänderung des Oberschenkelkopfes als Funktion einer Position und/oder Orientierung des mindestens einen Knochenänderungswerkzeugs (204) mit Bezug auf das Knochenmodell des Oberschenkelkopf und -halses und ein Werkzeuggeometriemodell, mindestens vor Durchführung der Wiederherstellung, bestimmt.

2. Computergestütztes System zur Hüftgelenkwiederherstellung (200) nach Anspruch 1, wobei das mindestens eine Knochenänderungswerkzeug (204) ein Bohrer ist, der bereitgestellt ist, um einen Führungskanal im Oberschenkelkopf herzustellen, um anschließend ein Wiederherstellungswerkzeug zu führen, wobei eine Orientierung des Bohrers vom Wiederherstellungsauswerter benutzt wird, um die ausgewertete Knochenwiederherstellungsänderung durch das Wiederherstellungswerkzeug als Funktion des Werkzeuggeometriemodells des Wiederherstellungswerkzeugs zu bestimmen.

3. Computergestütztes System zur Hüftgelenkwiederherstellung (200) nach Anspruch 1, ferner umfassend eine Anzeigeeinheit, die mit der Wiederherstellungs-Verarbeitungseinheit verbunden ist, um mit der ausgewerteten Knochenwiederherstellungsänderung assoziierte Daten visuell bereitzustellen.

4. Computergestütztes System zur Hüftgelenkwiederherstellung (200) nach Anspruch 3, wobei die mit der ausgewerteten Knochenwiederherstellungsänderung assoziierten Daten eine visuelle Echtzeitdarstellung des mit Bezug auf das Modell des Oberschenkelkopfes und -halses orientierten Werkzeuggeometriemodells sind.

5. Computergestütztes System zur Hüftgelenkwiederherstellung (200) nach Anspruch 4, wobei das mindestens eine Knochenänderungswerkzeug (204) ein Bohrer ist, der bereitgestellt ist, um einen Führungskanal im Oberschenkelkopf herzustellen, um anschließend ein Reibwerkzeug zu führen, wobei eine Orientierung des Bohrers vom Wiederherstellungsauswerter benutzt wird, um die ausgewertete Knochenwiederherstellungsänderung durch das Reibwerkzeug als Funktion des Werkzeuggeometriemodells des Reibwerkzeugs zu bestimmen, wobei das Reibwerkzeuggeometriemodell als parallele, mit Bezug auf das Modell des Oberschenkelkopfes und -halses orientierte Linien veranschaulicht ist.

6. Computergestütztes System zur Hüftgelenkwiederherstellung (200) nach Anspruch 1, wobei die Wiederherstellungs-Verarbeitungseinheit (201) einen chirurgischen Parameterkalkulator aufweist, um chirurgische Parameter mit Bezug auf den Oberschenkelreferenzrahmen, als Funktion von Verfolgungsdaten mindestens eines des Registrierungswerkzeugs (204), des mindestens einen Knochenänderungswerkzeugs (204) und zusätzlicher chirurgischer Werkzeuge, deren Position und Orientierung verfolgt wird, zu berechnen.

7. Computergestütztes System zur Hüftgelenkwiederherstellung (200) nach Anspruch 6, wobei die chirurgischen Parameter beliebige der Position des Drehmittelpunkts des Oberschenkelkopfes, des Varus/Valgus-Winkels, des Anteversionswinkels, der Gliedlängendiskrepanz beinhalten.

8. Computergestütztes System zur Hüftgelenkwiederherstellung nach Anspruch 1, wobei der Modellgenerator das Modell des Oberschenkelkopfes und -halses durch Ermitteln einer vorbestimmten Menge von Punkten auf dem Oberschenkelkopf und -hals durch das Verfolgen des Registrierungswerkzeugs erzeugt.

9. Computergestütztes System zur Hüftgelenkwiederherstellung nach Anspruch 1, wobei die Wiederherstellungs-Verarbeitungseinheit (201) ein vorgekerbtes Warnsystem umfasst, um das Kerben des Oberschenkelhalses zu vermeiden.

10. Verfahren zur Durchführung einer chirurgischen Behandlung mit einem computergestützten System zur Hüftgelenkwiederherstellung nach einem der Ansprüche 1-9 zum Führen eines Operateurs beim Wiederherstellen eines Oberschenkelkopfes bei einem anschließenden Implantieren eines Oberschenkelkopfimplantats, wobei das Verfahren an einem anatomischen Knochenmodell oder an einem Kadaver durchgeführt wird, wobei das Verfahren die folgenden Schritte umfasst:

   Definieren (104, 106, 108) eines Referenzrahmens des Oberschenkels, wobei der Referenzrahmen räumlich verfolgbar ist;
   Erzeugen (118) eines Modells eines Oberschenkelkopfes und -halses mit Bezug auf den Referenzrahmen;
   Auswählen (120) einer Orientierung eines Knochenänderungswerkzeugs mit Bezug auf das Modell des Oberschenkelkopfes und -halses als Funktion einer ausgewerteten Knochenwiederherstellungsänderung des Oberschenkelkopfes, das mit der Orientierung des Knochenänderungswerkzeugs variiert; und
   Herstellen (122) eines Führungskanals im Oberschenkelkopf mit dem Knochenänderungswerkzeug in der ausgewählten Orientierung, zum anschließenden Wiederherstellen des Oberschenkelkopfes.

11. Verfahren nach Anspruch 10, wobei das Modell des Oberschenkelkopfes und -halses durch Digitalisieren einer Oberfläche des Oberschenkelkopfes und -halses erzeugt wird.

12. Verfahren nach Anspruch 10, wobei die ausgewertete Knochenwiederherstellungsänderung eine visuelle Echtzeitdarstellung einer Geometrie eines Wiederherstellungswerkzeugs mit Bezug auf das Modell des Oberschenkelkopfes und -halses ist.

13. Verfahren nach Anspruch 10, ferner umfassend einen Schritt des Ermittelns chirurgischer Parameter beim Auswählen der Orientierung des Knochenänderungswerkzeugs und beim Herstellen des Führungskanals.

14. Verfahren nach Anspruch 13, wobei die chirurgischen Parameter beliebige der Position des Drehmittelpunkts des Oberschenkelkopfes, des Varus/Valgus-Winkels, des Anteversionswinkels, der Gliedlängendiskrepanz beinhalten.

15. Verfahren nach Anspruch 10, wobei das Herstellen (122) eines Führungskanals im Oberschenkelkopf das Warnen dort, wo potenzielles Kerben des Oberschenkelhalses auftreten könnte, umfasst.

## Revendications

1. Un système CAO de resurfaçage de hanche (200) pour guider un opérateur à resurfacer une tête fémorale en chirurgie assistée par ordinateur (CAO) pour implantation subséquente d'un implant de tête fémorale, comprenant:

   une référence traçable (202) sur le fémur, la référence traçable étant traçable pour former un cadre de référence fémoral du fémur;
   un outil d'enregistrement (204) étant traçable;
   au moins un outil d'altération d'os (204) associé à un resurfaçage de la tête fémorale, l'au moins un outil d'altération d'os étant traçable;
   un appareil de traçage (206) pour tracer la référence traçable (202), l'outil d'enregistrement (204) et l'au moins un outil d'altération d'os (204); et
   une unité processeur de resurfaçage (201) connectée à l'appareil de suivi (206) afin de recevoir des données de traçage pour la référence traçable (202), l'outil d'enregistrement (204) et l'au moins un outil d'altération d'os (204), l'unité processeur de resurfaçage ayant:

      un calculateur de position/orientation (212) pour calculer à partir des données de traçage une position et/ou orientation de la référence traçable (202) pour tracer le cadre de référence fémoral, et de l'outil d'enregistrement (204) et de l'au moins un outil d'altération d'os (204);
      un générateur de modèle recevant des données de position et/ou d'orientation de l'outil d'enregistrement (204) pour produire un modèle de la tête et du col fémoraux par rapport audit cadre de référence fémoral; et
      un évaluateur de resurfaçage (218) déterminant une altération évaluée de resurfaçage osseux de la tête fémorale en fonction d'une position et/ou d'une orientation de l'au moins un outil d'altération d'os (204) par rapport au modèle osseux de la tête et du col fémoraux, et un modèle géométrique d'outil, au moins avant l'exécution du resurfaçage.

2. Le système CAO (200) de resurfaçage de hanche selon la revendication 1, **caractérisé en ce que** l'au moins un outil d'altération d'os (204) est une perceuse fournie pour effectuer un canal de guidage dans la tête fémorale pour subséquemment guider un outil de resurfaçage, une orientation de la perceuse étant utilisée par l'évaluateur de resurfaçage pour déterminer l'altération évaluée de resurfaçage osseux par l'outil de resurfaçage en fonction du modèle géométrique d'outil de l'outil de resurfaçage.

3. Le système CAO (200) de resurfaçage de hanche selon la revendication 1, comprenant en outre une unité d'affichage connectée à l'unité processeur de resurfaçage pour fournir visuellement des données associées à l'altération évaluée de resurfaçage osseux.

4. Le système CAO (200) de resurfaçage de hanche selon la revendication 3, **caractérisé en ce que** les données associées à l'altération évaluée de resurfaçage osseux sont une représentation visuelle en temps réel du modèle géométrique d'outil orienté par rapport audit modèle de la tête et du col fémoraux.

5. Le système CAO (200) de resurfaçage de hanche selon la revendication 4, **caractérisé en ce que** l'au moins un outil d'altération d'os (204) est une perceuse fournie pour effectuer un canal de guidage dans la tête fémorale pour subséquemment guider un outil d'alésage, une orientation de la perceuse étant utilisée par l'évaluateur de resurfaçage pour déterminer l'altération évaluée de resurfaçage osseux par l'outil d'alésage en fonction du modèle géométrique d'outil de l'outil d'alésage, le modèle géométrique de l'outil d'alésage étant illustré en tant que lignes parallèles orientées par rapport au modèle de la tête et du col fémoraux.

6. Le système CAO (200) de resurfaçage de hanche selon la revendication 1, **caractérisé en ce que** l'unité processeur de resurfaçage (201) a un calculateur de paramètres chirurgicaux pour calculer des paramètres chirurgicaux par rapport audit cadre de référence fémoral, en fonction des données de suivi d'au moins l'un de l'outil d'enregistrement (204), l'au moins un outil d'altération d'os (204), et d'outils chirurgicaux supplémentaires tracés pour la position et l'orientation.

7. Le système CAO (200) de resurfaçage de hanche selon la revendication 6, **caractérisé en ce que** les paramètres chirurgicaux incluent l'un quelconque de la position du centre de rotation de la tête fémorale, un angle varus/valgus, un angle d'antéversion, un écart de longueur de membre.

8. Le système CAO (200) de resurfaçage de hanche selon la revendication 1, **caractérisé en ce que** le générateur de modèle produit le modèle de la tête et du col fémoraux en obtenant un nombre prédéterminé de points sur la tête et le col fémoraux par le traçage de l'outil d'enregistrement.

9. Le système CAO (200) de resurfaçage de hanche selon la revendication 1, **caractérisé en ce que** l'unité processeur de resurfaçage (201) comprend un système d'avertissement de pré-entaille afin d'éviter un entaillage du col du fémur.

10. Un procédé de réalisation de traitement chirurgical avec un système CAO de resurfaçage de hanche selon l'une quelconque des revendications 1 à 9 pour guider un opérateur à resurfacer une tête fémorale pour implantation subséquente d'un implant de tête fémorale, **caractérisé en ce que** le procédé est exécuté sur un modèle anatomique d'os ou sur un cadavre, le procédé comprenant les étapes de:

   définir (104, 106, 108) un cadre de référence du fémur, le cadre de référence pouvant être tracé dans l'espace;
   produire (118) un modèle de tête et de col fémoraux par rapport au cadre de référence;
   sélectionner (120) une orientation d'un outil d'altération d'os par rapport au modèle de la tête et du col fémoraux en fonction d'une altération évaluée de resurfaçage osseux de la tête fémorale variant avec ladite orientation de l'outil d'altération d'os; et
   créer (122) un canal de guidage dans la tête fémorale avec l'outil d'altération d'os dans l'orientation sélectionnée, pour un resurfaçage subséquent de la tête fémorale.

11. Le procédé selon la revendication 10, **caractérisé en ce que** le modèle de la tête et du col fémoraux est produit en numérisant une surface de la tête et du col fémoraux.

12. Le procédé selon la revendication 10, **caractérisé en ce que** l'altération évaluée de resurfaçage osseux est une représentation visuelle en temps réel d'une géométrie d'un outil de resurfaçage par rapport au modèle de la tête et du col fémoraux.

13. Le procédé selon la revendication 10, comprenant en outre une étape d'obtenir des paramètres chirurgicaux lors de la sélection de l'orientation de l'outil d'altération d'os et lors de la création du canal de guidage.

14. Le procédé selon la revendication 13, **caractérisé en ce que** les paramètres chirurgicaux incluent l'un quelconque de la position du centre de rotation de la tête fémorale, un angle varus/valgus, un angle d'antéversion, un écart de longueur de membre.

15. Le procédé selon la revendication 10, **caractérisé en ce que** la création (122) d'un canal de guidage dans la tête fémorale comprend avertir où l'entaillage potentiel du col fémoral pourrait se produire.

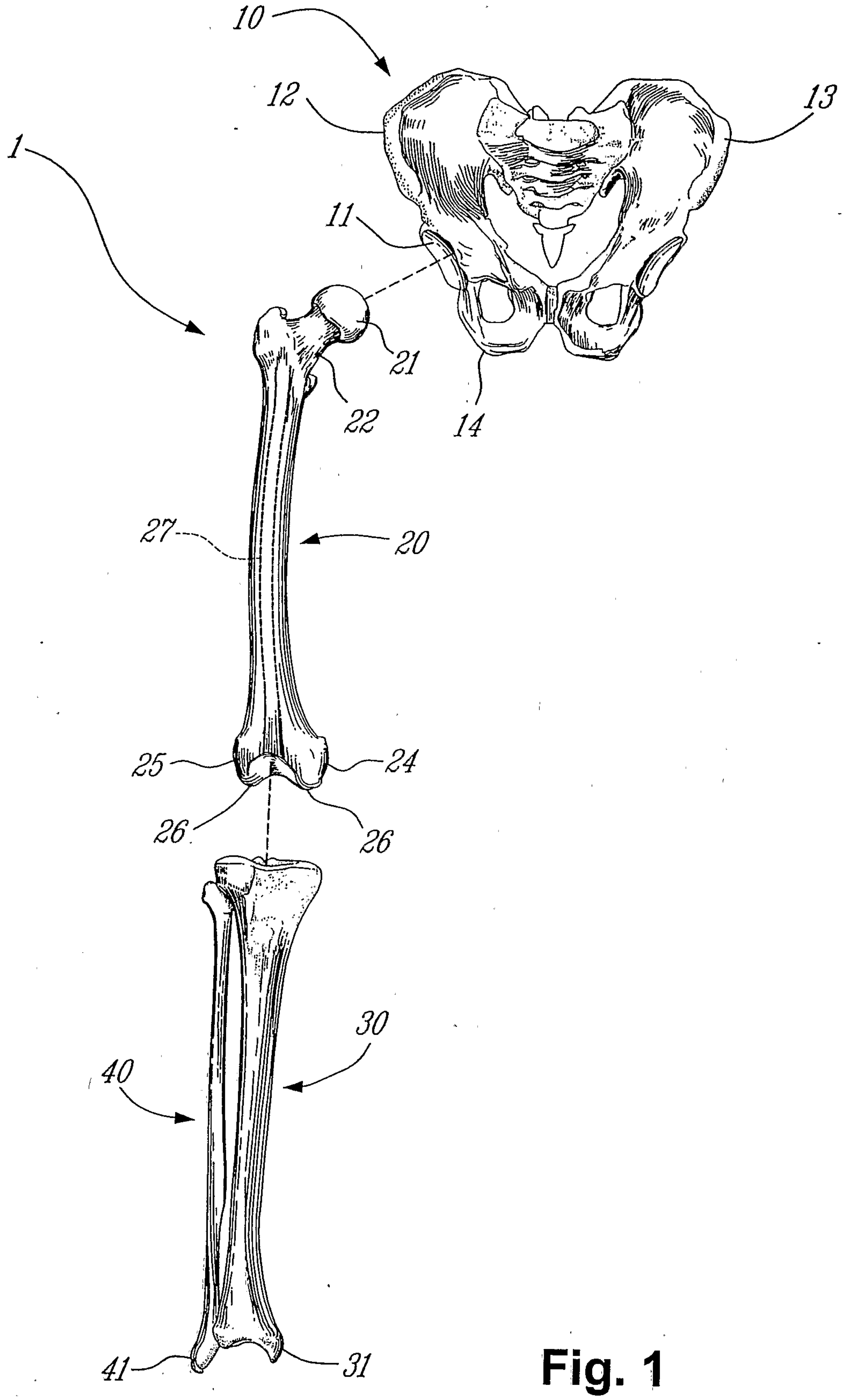


Fig. 1

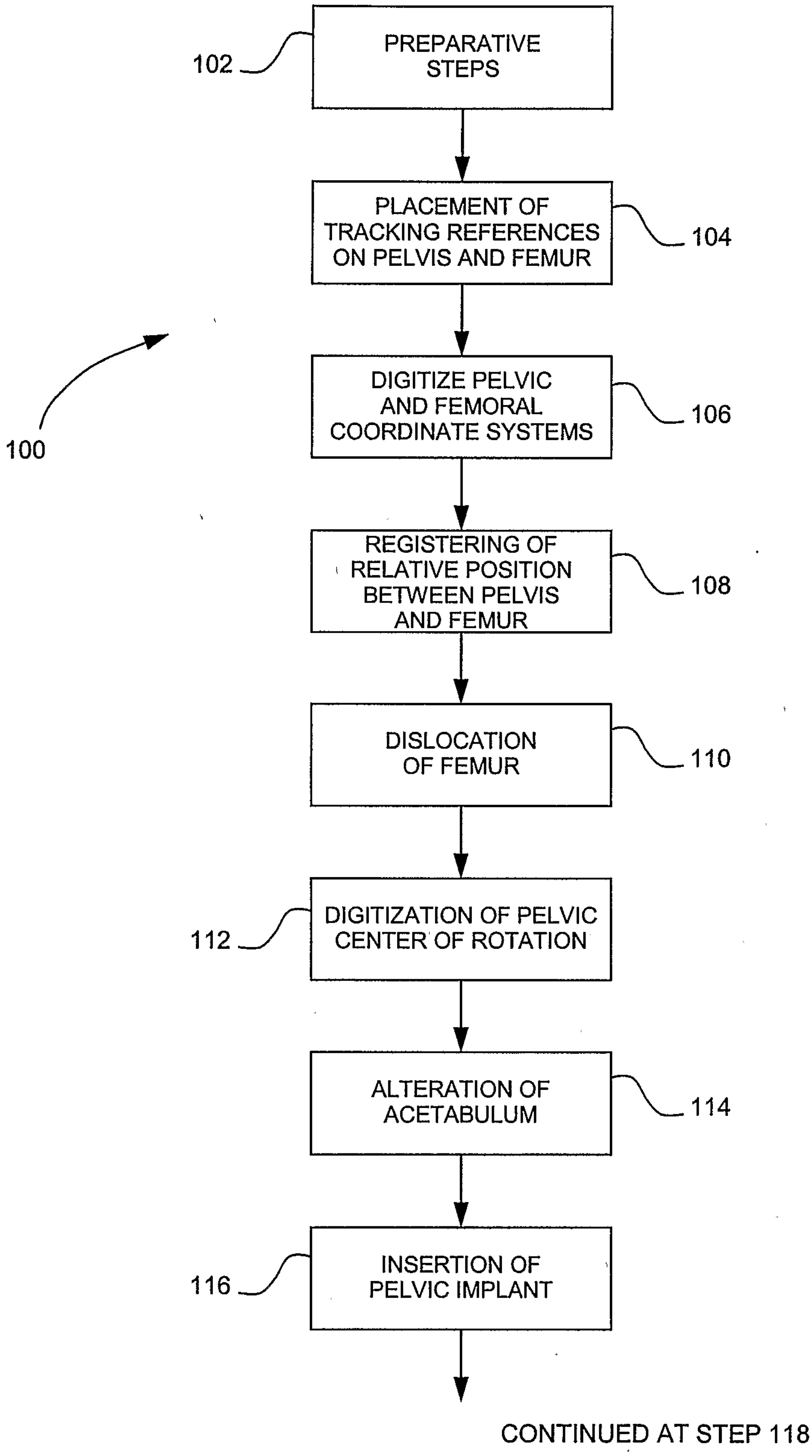
PREPARATIVE STEPS
102
PLACEMENT OF TRACKING REFERENCES ON PELVIS AND FEMUR
104
DIGITIZE PELVIC AND FEMORAL COORDINATE SYSTEMS
106
100
REGISTERING OF RELATIVE POSITION BETWEEN PELVIS AND FEMUR
108
DISLOCATION OF FEMUR
110
DIGITIZATION OF PELVIC CENTER OF ROTATION
112
ALTERATION OF ACETABULUM
114
INSERTION OF PELVIC IMPLANT
116
CONTINUED AT STEP 118

Fig. 2

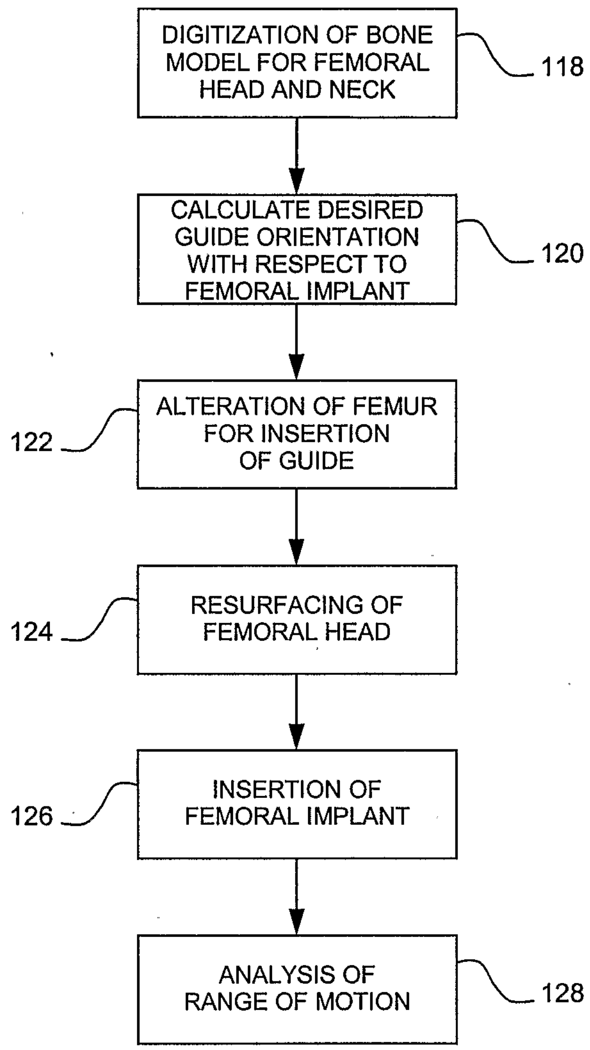
DIGITIZATION OF BONE MODEL FOR FEMORAL HEAD AND NECK
118
CALCULATE DESIRED GUIDE ORIENTATION WITH RESPECT TO FEMORAL IMPLANT
120
ALTERATION OF FEMUR FOR INSERTION OF GUIDE
122
RESURFACING OF FEMORAL HEAD
124
INSERTION OF FEMORAL IMPLANT
126
ANALYSIS OF RANGE OF MOTION
128

Fig. 2 (cont'd)

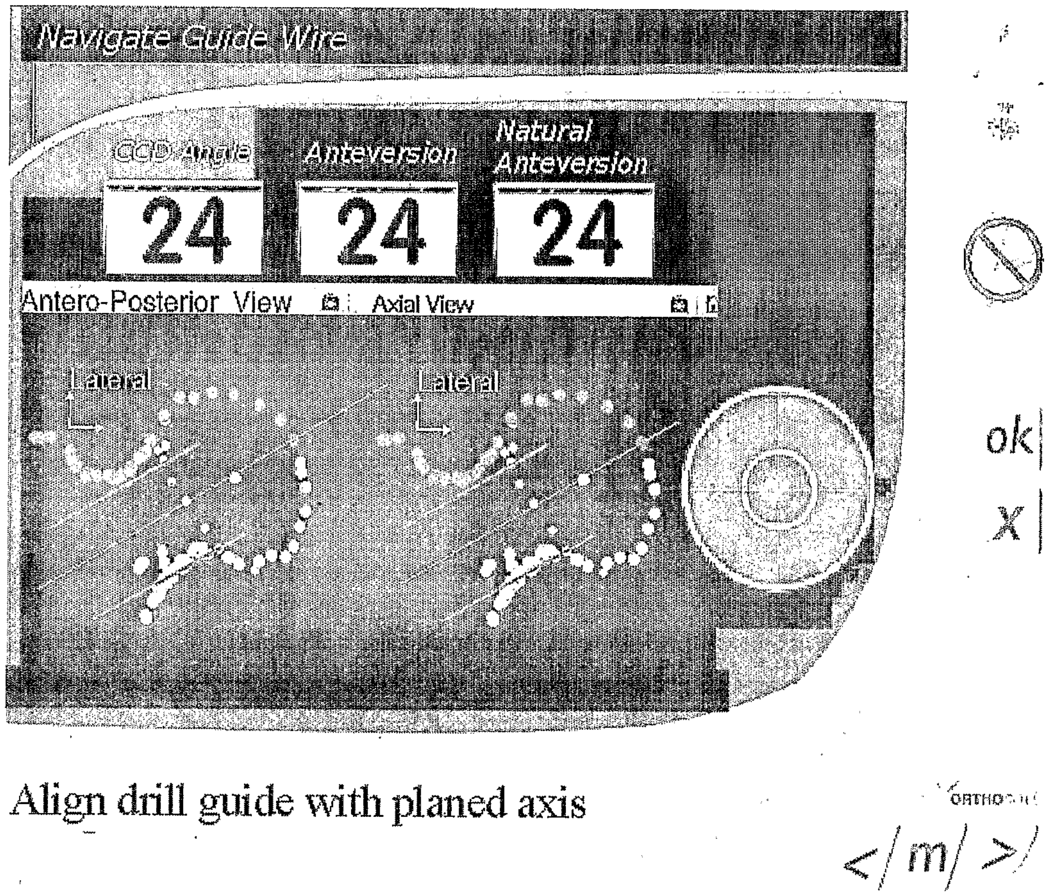
Navigate Guide Wire
CCD Angle
Anteversion
Natural Anteversion
24
24
24
Antero-Posterior View
Axial View
Lateral
Lateral
ok
x
Align drill guide with planed axis
ORTHO
</m/>

Fig. 3

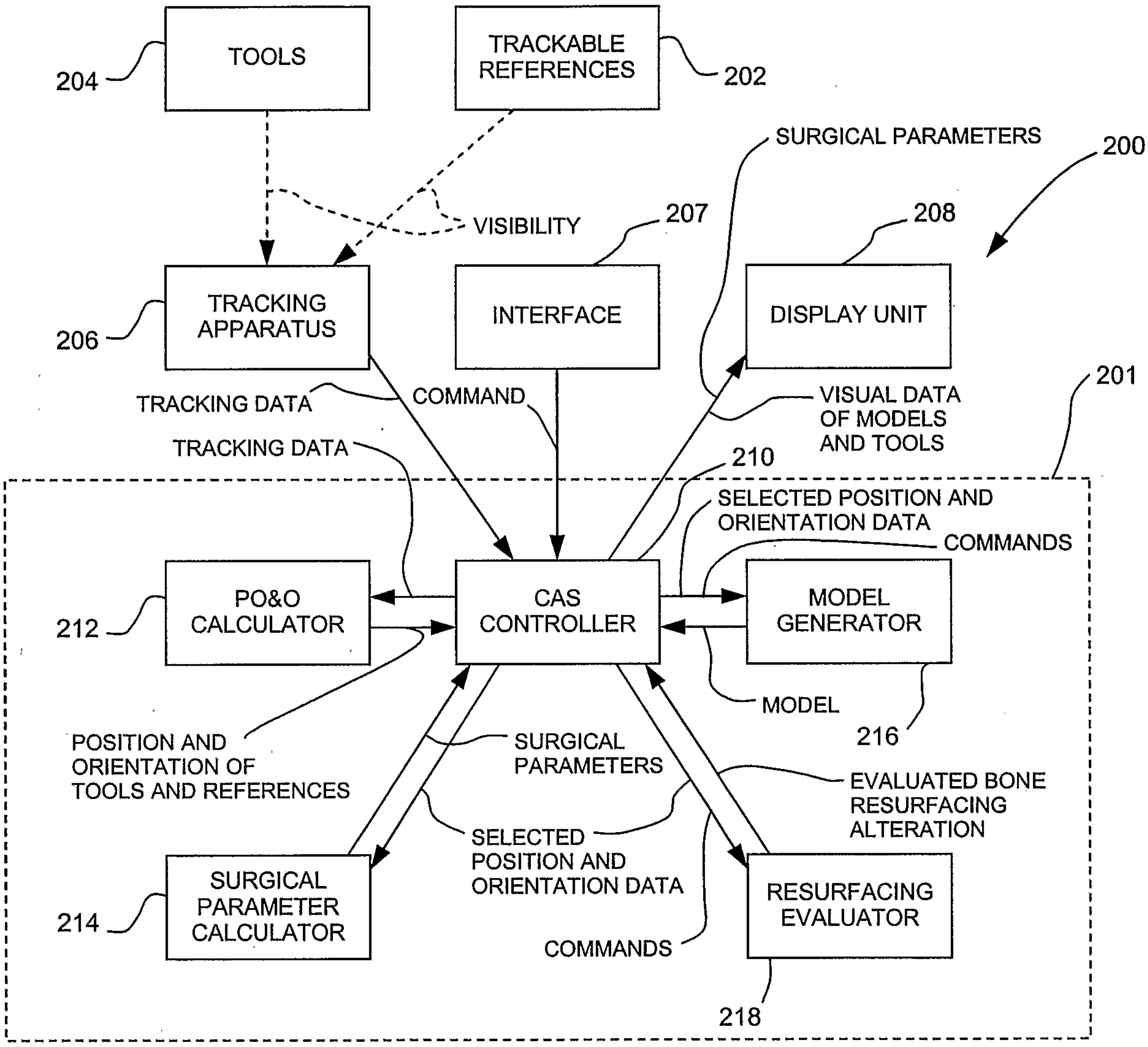
TOOLS
204
TRACKABLE REFERENCES
202
SURGICAL PARAMETERS
200
VISIBILITY
207
208
TRACKING APPARATUS
206
INTERFACE
DISPLAY UNIT
201
TRACKING DATA
COMMAND
VISUAL DATA OF MODELS AND TOOLS
TRACKING DATA
210
SELECTED POSITION AND ORIENTATION DATA
COMMANDS
PO&O CALCULATOR
212
CAS CONTROLLER
MODEL GENERATOR
MODEL
216
POSITION AND ORIENTATION OF TOOLS AND REFERENCES
SURGICAL PARAMETERS
EVALUATED BONE RESURFACING ALTERATION
SELECTED POSITION AND ORIENTATION DATA
SURGICAL PARAMETER CALCULATOR
214
RESURFACING EVALUATOR
COMMANDS
218

Fig. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004030556 A
- WO 03061501 A
- WO 0167979 A1, Jutras
- WO 2005102202 A
- WO 2004030559 A1, Jansen